(19) Europäisches Patentamt
European Patent Office
Office européen des brevets

(11) Veröffentlichungsnummer: **0 416 396 A2**

# (12) EUROPÄISCHE PATENTANMELDUNG

(21) Anmeldenummer: **90116231.3**

(22) Anmeldetag: **24.08.90**

(51) Int. Cl.5: **C07H 15/18**, C07H 13/04, //C07H3/06

(30) Priorität: **04.09.89 DE 3929295**

(43) Veröffentlichungstag der Anmeldung: **13.03.91 Patentblatt 91/11**

(84) Benannte Vertragsstaaten: **AT BE CH DE DK ES FR GB IT LI NL SE**

(71) Anmelder: **MERCK PATENT GESELLSCHAFT MIT BESCHRÄNKTER HAFTUNG**
**Frankfurter Strasse 250**
**W-6100 Darmstadt(DE)**

(72) Erfinder: **Kunz, Horst, Prof. Dr.**
**Gemeindehohl 50**
**W-6500 Mainz 33(DE)**
Erfinder: **Klinkhammer, Uwe, Dr.**
**Hans Böckler Strasse 53**
**W-6500 Mainz 1(DE)**
Erfinder: **Kinzy, Willy, Dr.**
**Beethovenstrasse 31**
**W-6845 Gross-Rohrheim(DE)**
Erfinder: **Neumann, Siegfried, Dr.**
**Battenbergstrasse 11**
**W-6104 Seeheim-Jugenheim(DE)**
Erfinder: **Radunz, Hans-Eckart, Dr.**
**Am Klingenteich 5**
**W-6109 Mühltal(DE)**

## (54) Tetrasaccharide und Verfahren zu ihrer Herstellung.

(57) Die Erfindung betrifft neue, vom Schizophyllan-Monomer ableitbare Tetrasaccharide, die eine intramolekulare Donor- und Akzeptorfunktion aufweisen und somit als Oligomerisierungsbaustein fungieren können, sowie ein Verfahren zu ihrer Herstellung.

EP 0 416 396 A2

## TETRASACCHARIDE UND VERFAHREN ZU IHRER HERSTELLUNG

Die Erfindung betrifft neue Tetrasaccharide, die in einem Molekül Donator- und Akzeptorfunktion aufweisen und somit als Oligomerisierungsbaustein geeignet sind, sowie ein neues Verfahren zur Herstellung dieser Tetrasaccharide einschließlich neuer Zwischenverbindungen.

Tetrasaccharide ähnlicher Struktur, jedoch ohne bifunktionellen Charakter, sind als "Repeating Units" des Schizophyllans bekannt (Ogawa und Kaburagi, Carbohydrate Research, 103 (1982), S. 53; Takeo und Tei, Carbohydrate Research, 145 (1986), S. 293). Schizophyllan selbst ist ein extrazelluläres Polysaccharid des Pilzes "Schizophyllum commune Fries" vom β-Glucan Typ.

Sowohl für das Polysaccharid als auch für einzelne Untereinheiten ist eine Antitumor-Wirkung nachgewiesen worden (z.B. Tabata et al., Carbohydrate Research, 89 (1981), S. 121). Verbindungen dieser Art sind daher als mögliche Antikrebsmittel oder Immunstimulanzien von Interesse.

Es bestand somit die Aufgabe, neue Verbindungen zur Verfügung zu stellen, die sich einerseits von der Repeating Unit des Schizophyllans ableiten, andererseits durch Einführung geeigneter funktioneller Gruppen auf einfache Art in der Weise aktivieren lassen, daß sie für gezielte, leicht handzuhabende Oligomerisierungsreaktionen eingesetzt werden können.

Es wurde nun gefunden, daß Tetrasaccharide der Formel I

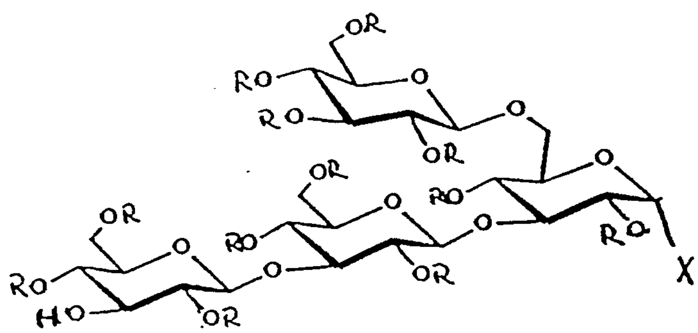

I

worin

X F, Cl, Br, $OC(=NH)CCl_3$ oder $SR^1$,

R H, $R^1CO$- oder $R^4CH_2$-,

$R^1$ Alkyl mit 1 bis 4 C-Atomen oder unsubstituiertes oder mit Halogen, OH oder Alkyl substituiertes Phenyl und

$R^4$ H oder Alkyl mit 1 bis 3 C-Atomen oder unsubstituiertes oder mit Halogen, OH, Alkyl oder O-Alkyl substituiertes Phenyl

bedeuten,

durch die Reste OH- (als Akzeptor) und X- (als Donator) an den entsprechenden Positionen des Tetrasaccharidbausteins in hervorragender Weise dazu geeignet sind, eine Polysaccharidkette gemäß der entsprechenden Tetrasaccharid-Untereinheit gezielt aufzubauen.

Die erfindungsgemäßen Verbindungen der Formel I lassen sich nach einem neuen Verfahren auf einfache Weise und in guten Ausbeuten durch Umsetzung von Disacchariden der Formel II (Donator),

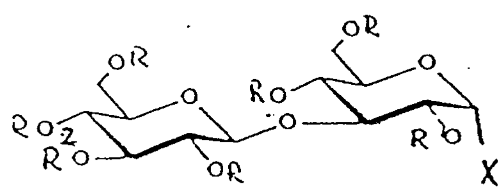

II

worin

$R^2$ $ZCH_2CO$-, $Z_2CHCO$- oder $Z_3CCO$- und

Z F oder Cl bedeuten und

X und R die angegebenen Bedeutungen haben,

bedeuten, mit Disacchariden der Formel III (Akzeptor),

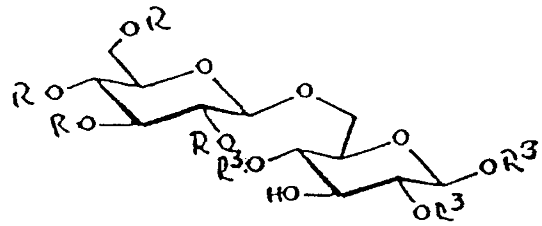

III

worin

$R^3$ $R^4CH_2$-

bedeutet, und $R^4$ die angegebene Bedeutung hat, über die Tetrasaccharide der Formel IV,

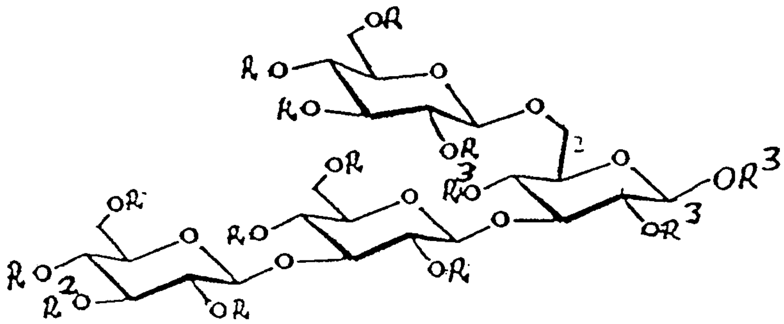

IV

worin R, $R^2$, $R^3$ die angegebenen Bedeutungen haben, erhalten.

Gegenstand der Erfindung sind somit Tetrasaccharide der Formel I.

Gegenstand der Erfindung ist außerdem ein Verfahren zur Herstellung von Tetrasacchariden der Formel I, dadurch gekennzeichnet, daß man Verbindungen der Formel II mit Verbindungen der Formel III zu den Tetrasacchariden der Formel IV umsetzt, die $OR^3$-Reste durch OR-Gruppen und die glycosidische Ester-Gruppe durch X substituiert und die $OR^2$-Gruppe hydrolysiert.

Gegenstand der Erfindung sind außerdem Disaccharide der Formel II.

Gegenstand der Erfindung sind weiterhin Disaccharide der Formel III.

Gegenstand der Erfindung sind ferner die Tetrasaccharide der Formel IV.

Gegenstand der Erfindung ist letztlich die Verwendung der Verbindungen der Formel I als Oligomerisierungsbaustein.

Vor- und nachstehend haben die Reste R, $R^1$, $R^2$, $R^3$, $R^4$, X und Z die angegebenen Bedeuturgen, sofern nicht ausdrücklich etwas anderes angegeben ist.

R bedeutet vorzugsweise $R^1$-CO-. Bedeutet $R^1$ Alkyl, so kann Alkyl geradkettig oder verzweigt sein. Im einzelnen sind dies Methyl, Ethyl, n-Propyl, Isopropyl, n-Butyl, Isobutyl, sec. und tert. Butyl. Vorzugsweise bedeutet Alkyl Methyl. Bedeutet $R^1$ substituiertes Phenyl, so kann Phenyl einfach oder mehrfach substituiert sein. Mit Ausnahme von Mesityl ist es aber vorzugsweise einfach substituiert. Als Substituenten eignen sich F, Cl, Br, OH oder Alkyl mit 1 bis 3 C-Atomen, vorzugsweise aber F, OH oder Methyl. $R^1$ bedeutet hier aber vorzugsweise $C_6H_5$-. Die im besonderen Maße bevorzugten Reste R sind demnach $CH_3CO$- und $C_6H_5CO$-.

$R^2$ bedeutet vorzugsweise $ZCH_2CO$, wobei Z F oder Cl, vorzugsweise Cl bedeutet. Besonders geeignet ist somit der Monochloracetyl-Rest.

X bedeutet vorzugsweise F. Bedeutet X $SR^1$, so ist $R^1$ bevorzugt substituiertes oder unsubstituiertes Phenyl, Methyl oder Ethyl.

$R^3$ bedeutet vorzugsweise Benzyl oder am Phenylring mit Alkyl oder O-Alkyl substituiertes Benzyl. Die im besonderen Maße bevorzugte Bedeutung von $R^4$ ist Phenyl oder mit Alkyl oder O-Alkyl substituiertes Phenyl.

Die Bedeutungen für einen bestimmten Rest innerhalb eines Moleküls können gleich oder verschiedenartig sein. Vorzugsweise sind sie gleich.

Die Herstellung der erfindungsgemäßen Tetrasaccharide der Formel I sowie der Zwischenverbindungen der Formeln II, III und IV erfolgt vorzugsweise nach dem Syntheseschema der Abb. 1. Ausgangsverbindungen sind vorzugsweise die leicht zugänglichen und im Handel erhältlichen Substanzen Diacetonglucose (DAG) und Pentacetyl-α-D-Glucose (PAG). Die nachfolgend aufgeführten einzelnen Syntheseschritte erfolgen, sofern nicht anders angegeben, nach in der Kohlenhydratchemie üblichen Standardmethoden. Die wichtigsten Synthesebausteine sind in Abb. 2 dargelegt.
Die Ziffern 1-12 bedeuten die wichtigsten in Abb. 2 klassifizierten Synthesebausteine.
Die Bedeutung der Reste R, $R^2$, $R^3$ und X ist im Text angegeben, ALL = Allyl.

Herstellung des Synthesebausteins 4:

Die Umsetzung der noch freien 3-OH-Gruppe der Diacetonglucose (1) zu einer wieder leicht zu entfernenden Ether-Gruppe geschieht beispielsweise mit Allylhalogeniden, vorzugsweise Allylbromid unter alkylierenden Bedingungen wie z.B. in Anwesenheit von Natriumhydrid. Von dem entstehenden Allylether-Derivat werden unter sauren Bedingungen in an sich bekannter Weise die bei der Veretherung als Schutzgruppen fungierenden Isopropyliden-Reste wieder abgespalten. Die Abspaltung kann besonders vorteilhaft mit einem sauren Ionenaustauscher erfolgen. Die so erhaltene 3-O-Allyl-D-Glucopyranose wird anschließend nach Standardmethoden vollständig zum Synthesebaustein 2 verestert (R = $R^1CO$-) oder verethert (R = $R^4CH_2$-). Als Reagenzien zur Veresterung dienen die der Bedeutung R entsprechenden Carbonsäuren, Carbonsäuresalze, Säurehalogenide oder Carbonsäureanhydride. Im besonderen Maße eignet sich Acetat in Eisessig oder Acetanhydrid in Pyridin unter wasserfreien Bedingungen. Bevorzugter Synthesebaustein 2 ist somit die peracetylierte 3-O-Allyl-D-glucopyranose. Die Verbindungen 2 werden, nun zu den $\alpha$-Donor-Verbindungen 3 umgesetzt. Der glycosidische Rest wird dabei in an sich bekannter Weise (z.B. nach der Helfrich-Synthese) durch X ersetzt. Hierbei sind die jeweiligen Fluoride besonders bevorzugt. Die Halogenierung am anomeren C-Atom der Glucopyranose erfolgt in üblicher Weise mit Halogenwasserstoff in beispielsweise Eisessig oder Pyridin. Für die Fluorierung verwendet man bevorzugt Fluorwasserstoff in Pyridin (Olah's Reagens). Die Halogenierung ist bekanntermaßen stereospezifisch, so daß nahezu quantitativ die $\alpha$-Derivate entstehen. Bevorzugte Verbindung 3 ist das 2,4,6-Tri-O-acetyl-3-O-allyl-$\alpha$-D-glucopyranosylfluorid. Die $\alpha$-Halogenide (X = Halogen) bzw. die Thio- oder Trichloracetimidatglycoside der Verbindungen 3 werden anschließend mittels der bekannten "Koenigs-Knorr"-Reaktion oder nach dem Trichloroacetimidatverfahren oder mit thiophilen Alkylierungskatalysatoren für die Thioglycoside (X = $SR^1$) in an sich bekannter Weise in die entsprechenden $\beta$-($OR^3$-) Glycoside überführt. Geht man von den bevorzugten Fluoriden aus, so lassen sich Lewis-Säuren wie beispielsweise Bortrifluorid-Etherat einsetzen. Als Reagens dienen die entsprechenden Alkohole $R^3OH$. Nach selektiver Überführung der drei OR-Schutzgruppen in freie OH-Gruppen nach Standardmethoden, beispielsweise mit Natriumalkoholaten oder mit Ammoniak erhält man so den Synthesebaustein 4. Bevorzugte Verbindung ist das Benzyl-3-O-allyl-$\beta$-D-glucopyranosid.

Herstellung des Synthesebausteins 6:

Bevorzugte Ausgangsverbindung ist der Synthesebaustein 4. Die noch freien OH-Gruppen werden in an sich bekannter Weise verethert, vorzugsweise mit $R^3X$ ($R^3$ = gesättigtes Alkyl, Alkylphenyl) unter alkylierenden Bedingungen. Insbesondere eignen sich die entsprechenden Bromide. Die Allylether-Gruppe der so entstandenen $R^3$-2,4,6-tri-O-$R^3$-3-O-allyl-$\beta$-D-glucopyranoside wird nun selektiv nach Standardmethoden, vorzugsweise unter Katalyse von Rhodium-I-Salzen (Wilkinson-Katalysator) isomerisiert und gespalten. Man erhält so den Synthesebaustein 6. Bevorzugte Verbindung ist hierbei das Benzyl-2,4,6-tri-O-benzyl-$\beta$-D-glucopyranosid.

Herstellung des Synthesebausteins 10:

Die noch freie OH-Gruppe der Diacetonglucose (1) wird wie oben beschrieben mit $R^3X$ ($R^3$ = gesättigtes Alkyl, Alkylphenyl) verethert. Danach werden nach der bei der Herstellung des Bausteins 4 näher beschriebenen bekannten Methode die Isopropyliden-Gruppe entfernt und die freien OH-Reste zu OR-Resten verestert. Bevorzugte Verbindung des Synthesebausteins 8 ist die 1,2,4,6-Tetra-O-acetyl-3-O-benzyl-$\beta$-D-glucopyranose. Die Benzyl- bzw. $R^3O$-Gruppe wird nun hydrogenolytisch nach Standardmethoden, beispielsweise mit Palladium auf Aktivkohle gespalten. In das so erhaltene Derivat läßt sich der Rest $R^2$ beispielsweise über das entsprechende Säurehalo genid in üblicher Weise leicht und in guten Ausbeuten einführen. Bevorzugte Verbindung des so hergestellten Synthesebausteins 9 ist die 1,2,4,6-Tetra-O-acetyl-3-O-monochloracetyl-$\beta$-D-glucopyranose. Wie bereits beim entsprechenden Allylderivat geschildert, wird nun die OR-Gruppe am anomeren Kohlenstoff durch X substituiert und man erhält den Synthesebaustein 10. Bevorzugt ist das 2,4,6-Tri-O-acetyl-3-O-monochloracetyl-α-D-glucosepyranosylfluorid.

Herstellung des Synthesebausteins 5:

Ausgangsverbindung ist zweckmäßigerweise der Synthesebaustein 4. Dieser wird in an sich bekannter Weise beispielsweise mit Benzaldehyddimethylacetal unter katalytischer Wirkung von zum Beispiel p-Toluolsulfonsäure zum entsprechenden Di-O-benzylidenderivat umacetalisiert. Die noch freie OH-Gruppe wird anschließend, wie bereits beschrieben, als Alkyl- bzw. Alkylphenyl- (z.B. Benzyl) Ether geschützt. Zu der freien 6-OH-Gruppe gelangt man nun durch reduktive Ringöffnung in an sich bekannter Weise. Vorzugsweise wird hierfür $LiAlH_4/AlCl_3$ eingesetzt, aber auch andere Reduktionsmittel sind geeignet. Die bevorzugte Verbindung des sc synthetisierten Bausteins 5 ist das Benzyl-2,4-di-O-benzyl-3-O-allyl-β-D-glucopyranosid.

Herstellung des Synthesebausteins 7:

Ausgangsverbindung ist die im Handel erhältliche Pentaacetyl-α-D-glucose (PAG). Diese wird in bekannter Weise unter Lewis-Säure-Katalyse (zum Beispiel mit Bortrifluorid-Etherat) mit Halogenwasserstoffsäure am glycosidischen C-Atom halogeniert. Vorzugsweise wird Fluorwasserstoffsäure eingesetzt. Bevorzugte Verbindung ist das 2,3,4,6-Tetra-O-acetyl-α-D-glucopyranosylfluorid.

Herstellung des Synthesebausteins 11:

Die beiden Synthesebausteine 5 und 7 reagieren nun in an sich bekannter Weise in einer Glycosylierungsreaktion zum Disaccharid 11. Wie bereits geschildert, werden hierfür, insbesondere bei der bevorzugten Verwendung von Fluoriden als Donatorbausteine, Lewis-Säuren als Katalysator, beispielsweise Bortrifluorid-Etherat, eingesetzt. Bei Einsatz entsprechender Chloride oder Bromide als Donatoren wird vorzugsweise mit anderen Katalysatoren, wie zum Beispiel Silbersalzen, aktiviert. Bevorzugte Verbindung des so synthetisierten Bausteins 11 ist das Benzyl-2,4-di-O-benzyl-O-allyl-6-O-(2,3,4,6-tetra-O-acetyl-β-D-glucopyranosyl)-β-D-glucopyranosid.

Herstellung der Verbindungen der Formel III

Die Allylgruppe wird nun in an sich bekannter Weise abgespalten. Man verwendet hierfür beispielsweise Palladiumsalze unter zumeist schwach sauren Bedingungen. Auch der Einsatz von anderen Katalysatoren, wie zum Beispiel den Wilkinson-Katalysator, ist prinzipiell möglich. Alkyl- bzw. Alkylphenyl-Ether werden dabei nicht angegriffen. Die so hergestellten neuen Disaccharidbausteine der Formel III sind Ausgangsverbindungen der erfindungsgemäßen Tetrasaccharide der Formeln IV und I. Bevorzugte Verbindung der Formel III ist das Benzyl-2,4-di-O-benzyl-6-O-(2,3,4,6-tetra-O-acetyl-β-D-glucopyranosyl)-β-D-glucopyranosid.

Herstellung des Synthesebausteins 12

Die beiden Synthesebausteine 6 und 10 reagieren in an sich bekannter Weise in einer Glycosylierungsreaktion zum Disaccharid 12. Die hierfür notwendigen Bedingungen entsprechen denen, die für die Herstellung von 11 bereits dargelegt wurden. Bevorzugte Verbindung von 12 ist das Benzyl-2,4,6-tri-O-benzyl-3-O-(2,4,6-tri-O-acetyl-3-0-monochloracetyl-β-D-glucopyranosyl)-β-D-glucopyranosid.

Herstellung der Verbindungen der Formel II

Die erfindungsgemäßen Verbindungen der Formel II lassen sich aus dem entsprechenden Synthesebaustein 12 beispielsweise durch Überführung der $OR^3$-Reste in freie Hydroxylgruppen und anschließender Veresterung zu OR-Gruppen herstellen. Dies geschieht üblicherweise nach Standardmethoden je nach Bedeutung des Restes $R^3$, beispielsweise durch Etherspaltung, vorzugsweise durch Hydrogenolyse mit zum Beispiel Wasserstoff/Palladium und jeweils anschließender Veresterung der OH-Reste mit $R^1$-CO- in üblicher Weise. Prinzipiell werden dabei solche Verfahrensschritte bevorzugt, bei denen die Reste OR und $OR^2$ nicht angegriffen werden. Ist dies aber unumgänglich, so lassen sich die jeweiligen Reste in der Regel durch einfache zusätzliche Reaktionen wieder einführen. In einem letzten Schritt wird die glykosidische OR-Gruppe durch Halogen, vorzugsweise Fluorid, nach Standardverfahren, zum Beispiel mit HF/Pyridin, ersetzt. Die Verbindungen der Formel II, insbesondere die bevorzugten Fluoride, stellen gut aktivierbare Donorbausteine für weitere Glycosylierungen dar. Bevorzugte Verbindung der Formel II ist das 2,4,6-Tri-O-acetyl-3-O-(2,4,6-tri-O-acetyl-3-O-monochloracetyl-β-D-glucopyranosyl)-α-D-glucopyranosylfluorid.

Herstellung der Verbindungen der Formel IV

Die erfindungsgemäßen Verbindungen der Formeln II und III reagieren in an sich bekannter Glycosylierungsreaktion zum Tetrasaccharid der Formel IV. Die Reaktionsbedingungen sind bereits bei der Herstellung der Disaccharide 11 und 12 genannt worden. Bevorzugte Verbindung ist das Benzyl-2,4-di-O-benzyl-3-O-[2,4,6-tri-O-acetyl-3-O-(2,4,6-tri-O-acetyl-3-O-monochloracetyl-β-D-glucopyranosyl)-β-D-glucopyranosyl]-6-O-(2,3,4,6-tetra-O-acetyl-β-D-glucopyranosyl)-β-D-glucopyranosid.

Aus den Verbindungen der Formel IV läßt sich auf einfache Weise das freie Schizophyllan-Monomer herstellen ($R = R^2 = R^3 = H$). Hierzu werden beispielsweise alle OR-, $OR^2$- und $OR^3$-Gruppen mittels an sich bekannter Methoden schrittweise oder in einer Stufe durch OH ersetzt (z.B. Ogawa u. Kaburaji, l.c.).

Herstellung der Verbindungen der Formel I

Die $OR^3$-Gruppen der Verbindungen der Formel IV werden nach dem erfindungsgemäßen Verfahren durch OR-Gruppen ersetzt, falls R $R^1$CO- bedeutet. Dies geschieht nach in der Kohlenhydratchemie bekannten Standardmethoden. Beispielsweise können die $OR^3$-Reste hydrogenolytisch entfernt und die freien Hydroxylgruppen anschließend zu OR-Gruppen ($R = R^1$-CO-) verestert werden. Auch direkte nucleophile Substitutionen, wobei $R^1COO^-$ als Nucleophil fungiert, können gegebenenfalls vorgenommen werden. Falls möglich (wie z.B. für $R^3$ = Benzyl) kann auch eine Hydrierung der $OR^3$-Reste stattfinden. Die Hydrierung wird im erfindungsgemäßen Verfahren eingesetzt, da bei den hier erforderlichen Bedingungen (z.B. Pd oder Pd/C) die anderen Reste nicht angegriffen werden. Auch kombinierte Verfahrenstechniken sind geeignet.

Als nächstes wird die glycosidische OR-Gruppe durch X, vorzugsweise Fluorid, nach Standardmethoden, z.B. mit HF/Pyridin, ersetzt. In einem letzten Schritt wird die als selektiv abspaltbare Schutzgruppe fungierende $OR^2$-Gruppe durch eine freie OH-Gruppe ersetzt, wodurch die Verbindungen ihre Akzeptoreigenschaft erhalten. Dies geschieht in an sich bekannter Weise z.B. mit Thioharnstoff in Ethanol (z.B. T.W. Greene, Protective Groups in Organic Synthesis, Wiley and Sons, New York 198).

Bevorzugte Verbindung der erfindungsgemäßen Tetrasaccharide der Formel I ist das 2,4-Di-O-acetyl-[2,4,6-tri-O-acetyl-3-O-(2,4,6-tri-O-acetyl-β-D-glucopyranosyl)-β-D-glucopyranosyl]-6-O-(2,3,4,6-tetra-O-acetyl-β-D-glucopyranosyl)-α-D-glucopyranosylfluorid.

Das erfindungsgemäße Verfahren zeichnet sich durch durchweg gute bis sehr gute Ausbeuten aus. So liegen die Ausbeuten für die einzelnen stereospezifischen Syntheseschritte zwischen 65 und 98 %, vorzugsweise zwischen 75 und 85 %. Die Glycosylierungen liefern Ausbeuten zwischen 65 und 85 %, vorzugsweise zwischen 70 und 80 %. Von den erfindungsgemäßen Glycosyldonatoren sind die entsprechenden Fluoride in besonderen Maße geeignet. Sie haben neben der ausgezeichneten Langzeitstabilität (bei Raumtemperatur bis zu mehreren Jahren) auch den Vorteil der leichten Aktivierbarkeit mit billigen und gut zu handhabenden Katalysatoren, wie z.B. Bortrifluorid-Etherat. Auf eine Aktivierung mit teuren, giftigen und teilweise explosiven Silbersalzen wie Silbertrifluoracetat Silbercarbonat und Silberperchlorat kann verzichtet werden. Das bevorzugte erfindungsgemäße Verfahren ist somit preiswerter und umweltfreundlicher als vergleichbare bekannte Methoden.

Überdies haben die erfindungsgemäßen Verbindungen der Formel I den weiteren Vorteil, daß sie jeweils eine Akzeptor- und eine Donatorfunktion in einem Molekül aufweisen. Sie sind somit hervorragend für Oligomerisierungsreaktionen geeignet. Dadurch kann gezielt eine Polymerkette aufgebaut werden, die so gegebenenfalls auf ihre spezifische pharmakologische Wirkung untersucht werden kann.

Die Oligomerisierung stellt eine intermolekulare Glycosylierungsreaktion des Tetrasaccharids der Formel I dar. Die Glycosylierung wird dabei unter den für die Herstellung der Di- und Tetrasaccharide beschriebenen Bedingungen durchgeführt und führt zu ähnlich guten Ausbeuten an Oligosaccharid. Zu den ungeschützten Oligomerisierungsprodukten (R = H) gelangt man vorzugsweise von dieser Endstufe aus mit Methoden, die bereits für den ungeschützten Baustein der Formel IV beschrieben wurden.

Beispiel 1

Zu einer Lösung von 150 g Diacetonglucose in 900 ml absolutem THF gibt man langsam und unter Kühlung, Rühren und Stickstoffatmosphäre 19,8 g NaH (80%ig in Mineralöl). Anschließend wird bei Raumtemperatur 1 Stunde gerührt. Es entsteht eine graugrüne Lösung. Man fügt 21 g Tetrabutylammoniumbromid hinzu und tropft anschließend unter Kühlung 120 ml Allylbromid hinzu. Nach ca. 1 h ist die Reaktion beendet. Zur Vernichtung von überschüssigem NaH tropft man vorsichtig unter Kühlung Methanol hinzu. Dann wird im Vakuum bis fast zur Trockne eingeengt und der Rückstand zwischen Wasser und Ether verteilt. Die Wasserphase wird noch einmal mit Ether ausgeschüttelt, und die vereinigten Etherfraktionen werden getrocknet und dann im Vakuum eingeengt. Nach Destillation des Rohprodukts ergibt sich 1,2:5,6-Di-O-isopropyliden-3-O-allyl-α-D-glucofuranose.

Beispiel 2

In 130 ml destilliertem $H_2O$ werden 155 g des Produktes aus Beispiel 1 mit 310 g saurem Ionenaustauscher Amberlite IR-120 mehrere Stunden lang bei etwa 60 °C gerührt. Dann wird vom Ionenaustauscher abfiltriert, mit $H_2O$ nachgewaschen und die vereinigten wässrigen Fraktionen zur Trockne eingeengt. Das Rohprodukt wird aus Ethanol/Hexan umkristallisiert. Es entsteht 3-O-Allyl-D-glucopyranose.

Beispiel 3

530 ml Essigsäureanhydrid und 88,5 g wasserfreies Natriumacetat werden unter Rühren bis kurz unter Rückflußtemperatur erhitzt. Dann gibt man vorsichtig 90 g des Produktes aus Beispiel 2 in kleinen Portionen hinzu und kocht ca. 30 min unter Rückfluß. Anschließend wird der überwiegende Teil des Lösungsmittels abgedampft und der Rückstand auf Eis gegossen. Es wird mehrmals mit je 400 ml Methylenchlorid extrahiert und die vereinigten organischen Phasen anschließend nacheinander mit Wasser, gesättigter Natriumhydrogencarbonatlösung und wieder mit Wasser gewaschen. Nach Trocknung und Einengen im Vakuum kann das Rohprodukt aus Ethanol/Hexan umkristallisiert werden. Man erhält 1,2,4,6-TetraO-acetyl-3-O-allyl-β-D-glucopyranose.

Beispiel 4

In einem Teflongefäß werden bei ca. 0 °C 20 g des Produktes aus Beispiel 3 unter einer Argonatmosphäre vorgelegt. Man gibt nun schnell 60 ml einer 70%igen Lösung von Fluorwasserstoff in Pyridin hinzu und rührt mehrere Stunden bei Raumtemperatur. Nach Beendigung der Reaktion gießt man auf Eiswasser und extrahiert dreimal mit 200 ml Methylenchlorid. Die vereinigten organischen Phasen werden nacheinander mit 1 N Salzsäure, gesättigter Natriumhydrogencarbonatlösung und Wasser gewaschen. Trocknen und Einengen im Vakuum ergibt ein hellbraunes Öl, das durch Flashchromatographie weiter gereinigt wird. Laufmittel: Petrolether (PE):Essigsäureethylester (EE) = 5:1. Man erhält 2,4,6-Tri-O-acetyl-3-O-allyl-α-D-glucopyranosylfluorid.

Beispiel 5

In 25 ml absolutem Methylenchlorid werden unter einer Argon- oder Stickstoffatmosphäre 2,65 g des Produktes aus Beispiel 4 und 4,1 g Trimethylsilylbenzylether mit 3 g Molsieb 4Å gerührt. Dann werden 3,1 ml $BF_3$ . $Et_2O$ langsam zugegeben. Es wird bei Raumtemperatur mehrere Stunden gerührt. Nach Beendigung der Reaktion wird mit Methylenchlorid verdünnt und über Kieselgur abgesaugt. Nach Ausschütteln mit Hydrogencarbonat-Lösung und Waschen mit Wasser wird getrocknet und im Vakuum eingeengt. Das Rohprodukt wird durch Flashchromatographie mit PE:EE = 4:1 gereinigt. Man erhält Benzyl-2,4,6-tri-O-acetyl-3-O-allyl-β-D-glucopyranosid. Schmp.: 59 °C

Beispiel 6

In 270 ml absolutem Methanol werden 33,5 g des Produktes aus Beispiel 5 gelöst. Dazu gibt man 11 ml 1 M Natriummethylat-Lösung in Methanol und erhitzt etwa 1 Stunde unter Rückfluß. Nach dem Abkühlen wird mit saurem Ionenaustauscher Amberlite IR-120 neutralisiert und abfiltriert. Nach Einengen wird mit PE:EE = 3:2 eine Kieselgelfiltration durchgeführt. Das Rohprodukt wird aus Petrolether/Ether umkristallisiert. Man erhält Benzyl-3-O-allyl-β-D-glucopyranosid. Schmp.: 80-81 °C

Beispiel 7

13,5 g des Produktes aus Beispiel 6 und 12 ml Benzaldehyddimethylacetal werden mit 70 mg p-Toluolsulfonsäure in 75 ml DMF gelöst und mehrere Stunden bei etwa 60 °C und ca. 60 mbar am Rotationsverdampfer reagieren gelassen. Dann wird das DMF abgedampft, der Rückstand in $CH_2Cl_2$ aufgenommen und mit Bicarbonat-Lösung ausgeschüttelt. Nach Abdampfen des Methylenchlorids wird der weiße Rückstand mit 500 ml eiskaltem Wasser/Petrolether (1/1, v/v) geschüttelt. Der Rückstand wird abgesaugt und nacheinander mit eiskaltem Wasser und Petrolether gewaschen und anschließend aus Ethanol umkristallisiert. Man erhält Benzyl-3-O-allyl-4,6-di-O-benzyliden-β-D-glucopyranosid. Schmp.: 136 °C

Beispiel 8

In 80 ml frisch destilliertem DMF werden 8,55 g des Produktes aus Beispiel 7 gelöst unter Kühlung mit 1,2 g NaH (80%ig in Mineralöl) versetzt. Man rührt noch eine Stunde bei Raumtemperatur und tropft dann 4 ml Benzylbromid unter Kühlung hinzu. Die Reaktion ist nach einigen Stunden bei Raumtemperatur beendet (DC PE:PE=4.1). Zur Aufarbeitung wird mit Methanol vorsichtig versetzt und anschließend eingedampft. Der Rückstand wird in Methylenchlorid aufgenommen, mit Wasser ausgeschüttelt, getrocknet und im Vakuum eingedampft. Das Rohprodukt wird aus Ethanol umkristallisiert. Man erhält Benzyl-2-O-benzyl-3-O-allyl-4,6-di-O-benzyliden-β-D-glucopyranosid. Schmp.: 117 °C

Beispiel 9

In einer 1:1-Mischung von 100 ml absolutem Ether/Methylenchlorid werden 5 g des Produktes aus Beispiel 8 gelöst und mit 1,95 g $LiAlH_4$ versetzt. Diese Lösung wird zum Rückfluß erhitzt und dann wird eine Lösung von 4,3 g $AlCl_3$ in 65 ml absolutem Ether zugetropft und ca. 2 Stunden unter Rückfluß erhitzt. Zur Vernichtung von überschüssigem Reagenz werden dann je 20 ml Essigester und Wasser zugetropft. Nach dem Abkühlen wird über Kieselgur abgesaugt und mit Methylenchlorid nachgewaschen. Die vereinigten organischen Phasen werden mit Wasser gewaschen, getrocknet und im Vakuum eingedampft. Das Rohprodukt wird aus Ethanol umkristallisiert. Man erhält Benzyl-2,4-di-O-benzyl-3-O-allyl-β-D-glucopyranosid. Schmp.: 118 °C

Beispiel 10

In einem Teflongefäß werden unter einer Argon- oder Stickstoffatmosphäre 50 ml flüssiger HF bei etwa -78 °C vorgelegt. Dazu gibt man 70 g Pentaacetylglucose und erwärmt auf Raumtemperatur. Zur Aufarbeitung gießt man auf eine Eis/Methylenchlorid-Mischung, die man in einem Teflonscheidetrichter vorgelegt hat. Man extrahiert noch dreimal mit je 300 ml Methylenchlorid. Die vereinigten organischen Phasen werden nacheinander mit Natriumhydrogencarbonatlösung und Wasser gewaschen, getrocknet und im Vakuum eingeengt. Das entstehende Öl läßt sich aus Ethanol oder Ethanol/Wasser umkristallisieren. Man erhält 2,3,4,6-Tetra-O-acetyl-α-D-glucopyranosylfluorid. Schmp.: 105 °C

Beispiel 11

In 350 ml frisch destilliertem DMF werden 22,6 g des Produktes aus Beispiel 6 gelöst. Unter Eiskühlung und Stickstoffatmosphäre werden vorsichtig 9,66 g NaH (80%ig in Mineralöl) zugegeben und eine Stunde bei Raumtemperatur gerührt. Man kühlt wieder auf ca. 0 °C ab und tropft bei dieser Temperatur 39 ml Benzylbromid hinzu. Bei Raumtemperatur wird einige Stunden weitergerührt. Zur Aufarbeitung wird mit Methanol versetzt und anschließend im Ölpumpenvakuum eingeengt. Der Rückstand wird in Methylenchlorid aufgenommen und mit Wasser ausgeschüttelt. Nach Trocknung und Eindampfen erhält man ein Rohprodukt, das sich aus Ethanol umkristallisieren läßt. Es ergibt sich Benzyl-2,4,6-tri-O-benzyl-3-O-allyl-β-D-glucopyranosid. Schmp.: 91 °C

Beispiel 12

In 500 ml einer Mischung aus Ethanol, Toluol und Wasser (8:3:1, v/v/v) werden 20 g des Produktes aus Beispiel 11 gelöst. Dazu gibt man 3,34 g 1,4-Diazabicyclo[2.2.2]octan und 0,67 g Tris-(triphenylphosphin)-rhodium-I-chlorid und erhitzt einige Stunden unter Rückfluß. Dann wird eingeengt und der Rückstand in Methylenchlorid aufgenommen. Die organische Phase wird nacheinander mit 1 N Salzsäure, Natriumhydrogencarbonatlösung und Wasser ausgeschüttelt. Nach Trocknung und Einengen wird in 295 ml Aceton/1 N Salzsäure aufgenommen und 15 Minuten unter Rückfluß erhitzt. Zur Aufarbeitung schüttelt man mit Bicarbonat-Lösung aus, filtriert vom Niederschlag ab, wäscht mit Aceton nach und engt ein. Der Rückstand wird in Methylenchlorid aufgenommen und mit Wasser gewaschen. Nach Trocknung wird eingeengt und der Rückstand aus Ethanol umkristallisiert. Man erhält Benzyl-2,4,6-tri-O-benzyl-β-D-glucopyranosid. Schmp.: 30-33 °C

Beispiel 13

Zu einer Lösung von 80 g Diacetonglucose in 480 ml absolutem THF gibt man langsam unter Kühlung, Rühren und Stickstoffatmosphäre 10,6 g NaH (80%ig in Mineralöl). Anschließend wird bei Raumtemperatur gerührt. Es entsteht eine graugrüne Lösung. Man fügt 11,2 g Tetrabutylammoniumbromid hinzu, tropft anschließend unter Eiskühlung 40 ml Benzylbromid hinzu. Nach ca. 1 h ist die Reaktion beendet (DC PE:EE = 1:1). Zur Vernichtung von überschüssigem NaH tropft man vorsichtig unter Kühlung Methanol hinzu. Dann wird im Vakuum bis fast zur Trockne eingeengt und der Rückstand zwischen Wasser und Ether verteilt. Die Wasserphase wird noch einmal mit Ether ausgeschüttelt und die vereinigten Etherfraktionen werden getrocknet und dann im Vakuum eingeengt. Das Rohprodukt ist für weitere Umsetzungen rein genug. Man erhält 1,2:5,6-Di-O-isopropyliden-3-O-benzyl-α-D-glucofuranose.

Beispiel 14

99 g des Produktes aus Beispiel 13 werden in 1000 ml destilliertem $H_2O$ mit 200 g saurem Ionenaustauscher Amberlite IR-120 mehrere Stunden lang bei 60 °C gerührt. Dann wird vom Ionenaustauscher abfiltiert, mit $H_2O$ nachgewaschen und die vereinigten wäßrigen Fraktionen zur Trockne eingeengt. Das Rohprodukt wird aus Ethanol/Hexan umkristallisiert. Man erhält 3-O-Benzyl-D-glucopyranose. Schmp.: 127 °C

Beispiel 15

In frisch destilliertem Pyridin werden 67 g des Produktes aus Beispiel 14 gelöst und unter Eiskühlung mit 375 ml Essigsäureanhydrid versetzt. Bei Raumtemperatur wird ca. 2 Tage gerührt. Zur Aufarbeitung wird die Hauptmenge Pyridin abdestilliert und der Rückstand auf Eis gegossen und mehrmals mit Methylenchlorid extrahiert. Die vereinigten organischen Phasen werden nacheinander in 1 N Salzsäure, Natriumhydrogencarbonatlösung und Wasser gewaschen, getrocknet und eingeengt. Das Rohprodukt wird aus Ethanol umkristallisiert. Man erhält 1,2,4,6-Tetra-O-acetyl-3-O-benzyl-β-D-glucopyranose. Schmp.: 102 °C

Beispiel 16

Zu 78 g des Produktes aus Beispiel 15, gelöst in 390 ml Eisessig werden 23,4 g Pd/C (5 %) Hydrierkatalysator gegeben und 25-35 Stunden unter einer Wasserstoffatomosphäre gerührt. Nach Ende der Reaktion wird über eine Celit-Schicht abgesaugt, mit Methylenchlorid mehrmals nachgewaschen und im Vakuum eingeengt. Das Produkt läßt sich aus Ether/Petrolether umkristallisieren. Man erhält 1,2,4,6-Tetra-O-acetyl-β-D-glucopyranose. Schmp.: 124 °C

Beispiel 17

In 1000 ml absolutem Mehtylenchlorid werden 60 g des Produktes aus Beispiel 16 gelöst und mit 75 ml absolutem Triethylamin versetzt. Die Lösung wird auf ca. -15 °C abgekühlt und mit 75 ml Chloracetylchlorid in 500 ml absolutem Methylenchlorid versetzt. Die anfänglich farblose Lösung färbt sich während dieser Zeit dunkelbraun bis schwarz. Man rührt dann mehrere Tage bei Raumtemperatur weiter. Zur Aufarbeitung wird eingeengt und mehr mals mit Toluol kodestilliert. Daß schwarze Öl wird in Methylenchlorid aufgenommen und nacheinander mit 1 N Salzsäure, Bicarbonat-Lösung und Wasser gewaschen, getrocknet und eingeengt. Zur weiteren Reinigung wird eine Kieselgel-Filtration mit Petrolether:Essigester = 4:1 als Eluens durchgeführt. Nach Umkristallisation aus Ethanol erhält man farblose Kristalle von 1,2,4,6-Tetra-O-monochloracetyl-β-D-glucopyranose. Schmp.: 110 °C

Beispiel 18

In einem Teflongefäß werden unter einer Argonatmosphäre 50 ml HF/Pyridin-Lösung auf ca. -15 °C abgekühlt und dann mit 10 g des Produktes aus Beispiel 17 versetzt und im Eisbad gerührt. Nach Beendigung der Reaktion wird im Teflonscheidetrichter auf Eis gegossen und mehrmals mit Methylenchlorid extrahiert. Die vereinigten organischen Phasen werden nacheinander mit 1 N Salzsäure, Bicarbonat-Lösung und Wasser gewaschen, getrocknet und im Vakuum eingeengt. Man erhält nach Kristallisation aus Ethanol 2,4,6-Tri-O-acetyl-3-O-monochloracetyl-α-D-glucopyranosylfluorid. Schmp.: 93-94 °C

Beispiel 19

In einem ausgeheizten Kolben werden 100 mg Benzyl-2,4-di-O-benzyl-3-O-allyl-β-D-glucopyranosid und 100 mg 2,3,4,6-Tetra-O-acetyl-α-D-glucopyranosylfluorid im Ölpumpenvakuum getrocknet. In den mit trokkenem Argon belüfteten Kolben gibt man 200 mg mit dem Quarzstrahler ausgeheiztes feingepulvertes Molsieb 4Å und versetzt mit 5 ml frisch absolutiertem Methylenchlorid. Man rührt bei Raumtemperatur und gibt dann 130 mg frisch destilliertes $BF_3$ . $Et_2O$ zu. Man rührt über Nacht bei Raumtemperatur. Zur Aufarbeitung wird mit Methylenchlorid verdünnt und über Celit abgesaugt. Es wird mit Natriumhydrogencarbonat und Wasser gewaschen, getrocknet und im Vakuum eingeengt. Flash-Chromatographie (PE:EE = 6:1) liefert einen farblosen Schaum, der aus Ethanol umkristallisiert wird. Man erhält Benzyl-2,4-di-O-benzyl-3-O-allyl-6-O-(2,3,4,6-tetra-O-acetyl-β-D-glucopyransoyl)-β-D-glucopyranosid. Schmp.: 119 °C

Beispiel 20

Eine Mischung aus 2,3 g des Produktes aus Beispiel 19, Palladium-II-chlorid (0,7 g) und 0,7 g wasserfreiem Natriumacetat in 28 ml Eisessig-Wasser (20:1) wird einige Stunden bei Raumtemperatur gerührt. Nach Beendigung der Reaktion wird über Celit abgesaugt und mehrmals mit Methylenchlorid nachgewaschen. Die vereinigten Filtrate werden im Vakuum eingeengt und mittels Flash-Chromatographie (PE:EE = 6:1) weiter gereinigt. Das Rohprodukt wird aus Ethanol umkristallisiert. Man erhält Benzyl-2,4-di-O-benzyl-6-O-(2,3,4,6-tetra-O-acetyl-β-D-glucopyranosyl)-β-D-glucopyranosid. Schmp.: 82-84 °C

Beispiel 21

Analog Beispiel 19 werden 100 mg Benzyl-2,4,6-tri-O-benzyl-β-D-glucopyranosid und 100 mg 2,4,6-Tri-O-acetyl-3-O-monochloracetyl-α-D-glucopyranosylfluorid umgesetzt. Man erhält Benzyl-2,4,6-tri-O-benzyl-3-O-(2,4,6-tri-O-acetyl-3-O-monochloracetyl-β-D-glucopyranosyl)-β-D-glucopyranosid.

Beispiel 22

In frisch destilliertem Dioxan (50 ml) werden 860 mg des Produktes aus Beispiel 21 gelöst und mit 500 mg Pd/C (5 %) Hydrierkatalysator versetzt und über Nacht hydriert. Man saugt über Celit ab, wäscht mehrmals mit Methanol nach und engt im Vakuum ein. Zur Reinigung wird eine Flash-Chromatographie mit $CH_2Cl_2$:MeOH = 15:1 durchgeführt. Man erhält einen farblosen Schaum. Das Produkt wird anschließend sofort weiterverarbeitet. Dazu löst man es in 10 ml Essigsäureanhydrid, gibt 500 mg wasserfreies Natriumacetat hinzu und rührt über Nacht bei Raumtemperatur. Zur Aufarbeitung engt man ein, nimmt den Rückstand in Methylenchlorid auf und wäscht nacheinander mit Wasser, Bicarbonat-Lösung und wieder mit Wasser. Nach Trocknung und Einengen wird durch Flash-Chromatographie mit PE:EE = 2:1 gereinigt. Man erhält einen farblosen Schaum. Man erhält 1,2,4,6-Tetra-O-acetyl-3-O-(2,4,6-tri-O-acetyl-3-O-monochloracetyl-β-D-glucopyranosyl)-D-glucopyranose. In einem Teflongefäß werden 10 ml HF/Pyridin-Lösung unter Argon auf 0 °C abgekühlt, bei dieser Temperatur 4 g des erhaltenen Produktes zugegeben und ca. 60 Minuten bei dieser Temperatur gerührt. Zur Aufarbeitung gießt man auf Eis und extrahiert mehrmals mit Methylenchlorid. Die vereinigten organischen Phasen werden nacheinander mit verdünnter Salzsäure, Natriumhydrogencarbonat-Losung und Wasser gewaschen, getrocknet und im Vakuum eingeengt. Flash-Chromatographie (PE:EE = 2:1) liefert ein farbloses Gel, das bei längerem Stehen erstarrt. Man erhält 2,4,6-Tri-O-acetyl-3-O-(2,4,6-tri-O-acetyl-3-O-monochloracetyl-β-D-glucopyranosyl)-α-D-glucopyranosylfluorid.

Beispiel 23

Analog Beispiel 19 werden 1340 mg des Produktes aus Beispiel 20 und 960 mg des Produktes aus Beispiel 22 zur Glycosylierung umgesetzt. Man erhält Benzyl-2,4-di-O-benzyl-3-O-[2,4,6-tri-O-acetyl-3-O-(2,4,6-tri-O-acetyl-3-O-monochloracetyl-β-D-glucopyranosyl)-β-D-glucopyranosyl]-6-O-(2,3,4,6-tetra-O-acetyl-β-D-glucopyranosyl)-β-D-glucopyranosid.

Beispiel 24

Analog Beispiel 22 werden 1,25 g des Produktes aus Beispiel 23 umgesetzt. Man erhält 2,4-Di-O-acetyl-3-O-[2,4,6-tri-O-acetyl-3-O-(2,4,6-tri-O-acetyl-3-O-monochloracetyl-β-D-glucopyranosyl)-β-D-glucopyranosyl]-6-O-(2,3,4,6-tetra-O-acetyl-β-D-glucopyranosyl)-α-D-glucopyranosylfluorid.

Beispiel 25

310 mg des Produkts aus Beispiel 24 werden bei 0 °C in einer Mischung aus 1,8 ml 2,6-Lutidin und 0,6 ml Eisessig gelöst und bei dieser Temperatur mit 2 ml frisch hergestellter Hydrazindithiocarbonat-Lösung versetzt. Nach 10 Minuten kann aufgearbeitet werden. Man verdünnt mit Methylenchlorid, schüttelt mit Wasser verdünnter HCl und Bicarbonat-Lösung aus und reinigt mit Flash-Chromatographie (PE: EE = 2:1). Man erhält 2,4-Di-O-acetyl-3-O-[2,4,6-tri-O-acetyl-3-O-(2,4,6-tri-O-acetyl-β-D-glucopyranosyl)-β-D-glucopyranosyl]-6-O-(2,3,4,6-tetra-O-acetyl-β-D-glucopyranosyl)-α-D-glucopyranosylfluorid.

Darstellung der Hydrazindithiocarbonat-Lösung:

Zu einer gekühlten Lösung (0 °C) von Hydrazinhydrat (0,73 ml) und Diisopropylethylamin in Ethanol/Wasser (30 ml, 2:1, v/v) wird eine Lösung von $CS_2$ (0,7 ml) in Dioxan (6 ml) tropfenweise hinzugefügt (C.A.A. van Boeckel, T. Beetz, Tetrahedron Lett. 24 (1983) 3775-3778).

Beispiel 26

306 mg des Produktes aus Beispiel 23 werden in 5 ml absolutem Methanol gelöst und unter Kühlung mit 120 µl 1 M Natriummethylat-Lösung versetzt. Nach ca. drei Tagen Rühren bei Raumtemperatur wird mit schwach saurem Ionenaustauscher neutralisiert und anschließend abfiltriert und mit Methanol nachgewaschen. Nach Einengen im Vakuum wird das erhaltene Öl durch Flash-Chromatographie mit Methylenchlorid:Methanol = 2:1 gereinigt. Man erhält Benzyl-3-O-[3-O-(β-D-glucopyranosyl)-β-D-glucopyranosyl]-6-O-(β-D-glucopyranosyl)-2,4-di-O-benzyl-β-D-glucopyranosid. 145 mg dieses Produktes werden in 10 ml Eisessig gelöst und bei 8 bar mit 10 % Pd/C (150 mg) einige Tage hydriert. Dann wird über Celit abgesaugt und mehrmals mit Wasser gewaschen. Nach Einengen im Hochvakuum wird der Rückstand durch präparative Dickschicht mit n-Butanol: Isopropanol:Wasser = 10:5:4 gereinigt. Man erhält 3-O-[3-O-(β-D-glucopyranosyl)-β-D-glucopyranosyl]-6-O-(β-D-glucopyranosyl)-β-D-glucopyranose.

Erläuterungen zu den Abbildungen:

Abb. 1: Syntheseschema; die Ziffern 1-12 bedeuten die wichtigsten in Abb. 2 klassifizierten Synthesebausteine.
DAG = Diacetonglucose
PAG = Pentaacetyl-α-D-Glucose
Abb. 2.: Synthesebausteine; die Bedeutung der Reste R, $R^2$, $R^3$ und X ist im Text angegeben, ALL = Allyl

**Ansprüche**

1. Tetrasaccharide der Formel I

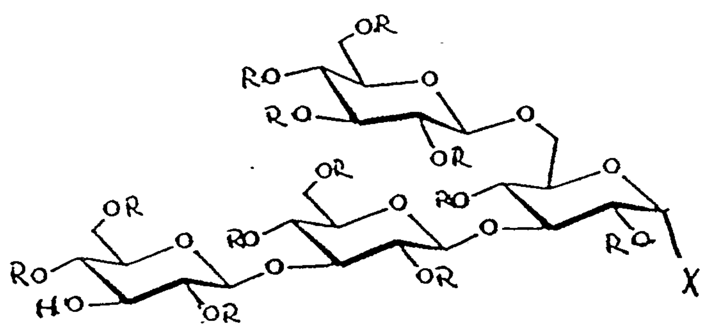

I

worin
X F, Cl, Br, $OC(=NH)CCl_3$ oder $SR^1$
R H, $R^1CO$- oder $R^4CH_2$-
$R^1$ Alkyl mit 1 bis 4 C-Atomen oder unsubstituiertes oder mit Halogen, OH oder Alkyl substituiertes Phenyl und
$R^4$ H oder Alkyl mit 1 bis 3 C-Atomen oder unsubstituiertes oder mit Halogen, OH, Alkyl oder O-Alkyl substituiertes Phenyl
bedeuten.
2. Verfahren zur Herstellung von Tetrasacchariden der Formel I, dadurch gekennzeichnet, daß man Verbindungen der Formel II

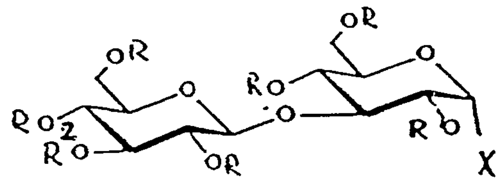

II

worin

$R^2$ $ZCH_2CO-$, $Z_2CHCO-$ oder $Z_3CCO-$ und
Z F oder Cl bedeuten und
X und R die angegebenen Bedeutungen haben
mit Verbindungen der Formel III

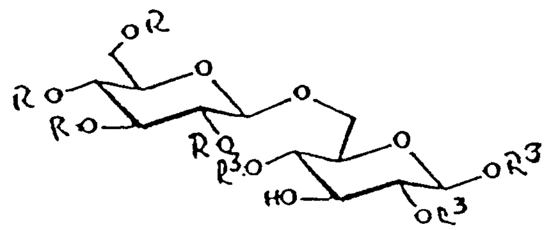

III

worin
$R^3$ $R^4CH_2-$
bedeutet und $R^4$ die angegebenen Bedeutungen hat,
zu den Tetrasacchariden der Formel IV

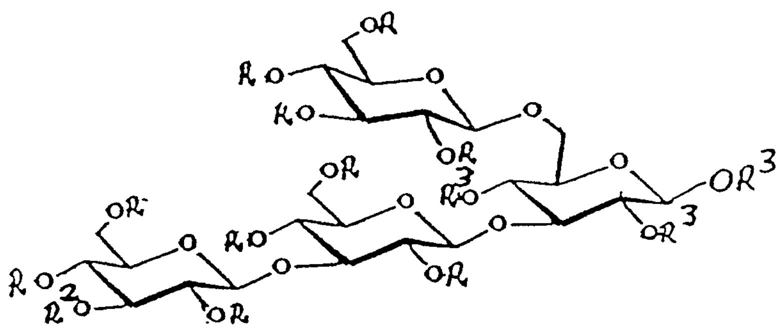

IV

worin
R, $R^2$ und $R^3$ die angegebenen Bedeutungen haben
umsetzt, die $OR^3$-Reste gegebenenfalls durch OR-Gruppen und die glycosidische Ester-Gruppe durch X substituiert und die $OR^2$-Gruppe hydrolysiert.

3. Disaccharide der Formel II, worin X, R und $R^2$ die angegebenen Bedeutungen haben.
4. Disaccharide der Formel III, worin R und $R^3$ die angegebenen Bedeutungen haben.
5. Tetrasaccharide der Formel IV, worin R, $R^2$ und $R^3$ die angegebenen Bedeutungen haben.
6. Verwendung der Verbindungen der Formel I als Oligomerisierungs-Baustein.

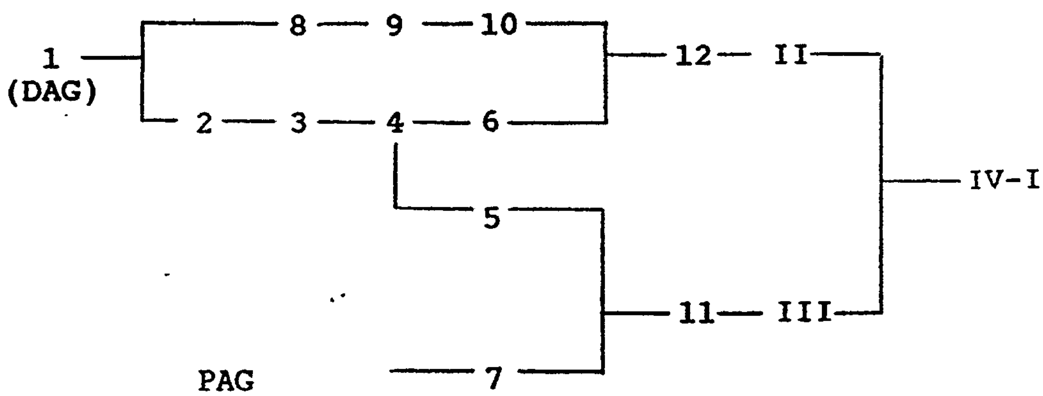


Abb. 1

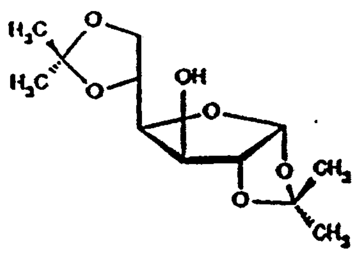

1

2

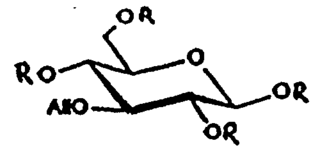

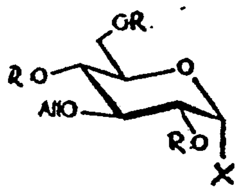

3

4

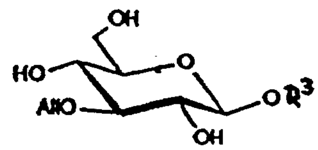

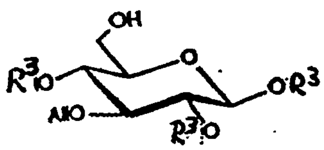

5

6

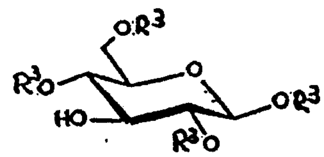

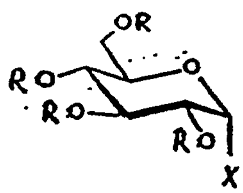

7

8

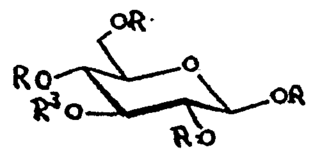

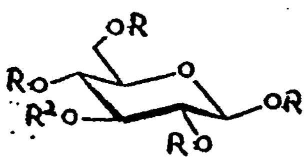

9

10

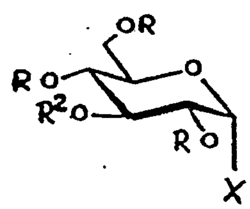

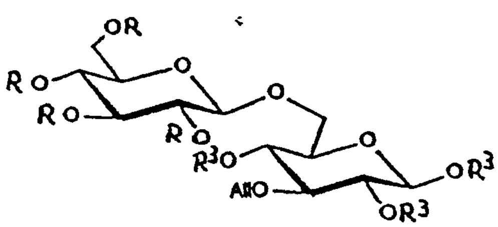

11

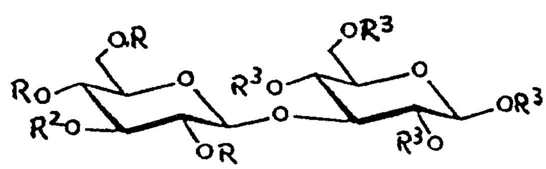

12

Abb. 2